# EUROPEAN PATENT APPLICATION

(11) **EP 2 017 619 A1**
(43) Date of publication of application: **21.01.2009**
(21) Application number: 07112839.1
(22) Date of filing: 20.07.2007
(51) Int. Cl.: G01N 33/543

(54) **Magnetic sensor device**

(71) Applicant: Koninklijke Philips Electronics N.V., 5621 BA Eindhoven (NL)
(72) Inventor: The designation of the inventor has not yet been filed
(74) Representative: Ledeboer, Johannes Albertus

(57) **Abstract**

The present invention provides a sensor device (20) and a method for determining the presence and/or amount of target moieties (22) in a sample fluid (27), the target moieties (22) being labeled with magnetic or magnetizable objects (23). The sensor device (20) comprises a magnetic field generating means adapted for applying a retention field for retaining the labeled target moieties (22, 23) to the sensor surface (24) during fluid manipulations. Through this the binding between the labeled target moieties (22, 23) and the sensor surface (24) and/or the magnetic particles (23) and the target moieties (22) is substantially not disturbed during these fluid manipulations. This leads to a reliable and accurate sensor device (20).

## Description

### FIELD OF THE INVENTION

The present invention relates to a magnetic sensor device and to a method for determining the presence and/or amount of target moieties in a sample fluid, the target moieties being labeled with magnetic or magnetizable objects, e.g. magnetic particles, as well as to a method of manufacturing the sensor device.

### BACKGROUND OF THE INVENTION

Magnetic sensors based on AMR (anisotropic magneto resistance), GMR (giant magneto resistance) and TMR (tunnel magneto resistance) elements or on Hall sensors, are nowadays gaining importance. Besides the known high-speed applications such as magnetic hard disk heads and MRAM, new relatively low bandwidth applications appear in the field of molecular diagnostics (MDx), current sensing in IC's, automotive, etc.

The introduction of micro-arrays or biochips comprising such magnetic sensors is revolutionizing the analysis of biomolecules such as DNA (desoxyribonucleic acid), RNA (ribonucleic acid) and proteins. Applications are, for example, human genotyping (e.g. in hospitals or by individual doctors or nurses), bacteriological screening, biological and pharmacological research. Such magnetic biochips have promising properties for, for example, biological or chemical sample analysis, in terms of sensitivity, specificity, integration, ease of use and costs.

Biochips, also called biosensor chips, biological microchips, gene-chips or DNA chips, consist in their simplest form of a substrate on which a large number of different probe molecules are attached, on well-defined regions on the chip, to which molecules or molecule fragments that are to be analyzed can bind if they are perfectly matched. For example, a fragment of a DNA molecule binds to one unique complementary DNA (c-DNA) molecular fragment. The occurrence of a binding reaction can be detected, for example by using markers, e.g. fluorescent markers or magnetic labels, which are coupled to the molecules to be analyzed, either before or after binding of these molecules to the probe molecules. This provides the ability to analyze small amounts of a large number of different molecules or molecular fragments in parallel, in a short time.

In a biosensor an assay takes place. Assays generally involve several fluid actuation steps, i.e. steps in which materials are brought into movement. Examples of such steps are mixing (e.g. for dilution, or for the dissolution of labels or other reagents into the sample fluid, or for labeling, or for affinity binding) or the refresh of fluid near to a reaction surface in order to avoid that diffusion becomes rate-limiting for the reaction. Preferably the actuation method should be effective, reliable and cheap.

One biochip can hold assays for 1000 or more different molecular fragments. It is expected that the usefulness of information that can become available from the use of biochips will increase rapidly during the coming decade, as a result of projects such as the Human Genome Project, and follow-up studies on the functions of genes and proteins.

A biosensor consisting of an array of, for example 100, sensors based on the detection of e.g. superparamagnetic beads may be used to simultaneously measure the concentration of a large number of different biological molecules (e.g. protein, DNA) in a solution (e.g. blood). This may be achieved by attaching a superparamagnetic bead to target molecules which are to be determined, magnetizing this bead with an applied magnetic field and using e.g. a GMR sensor to detect the magnetic field of the magnetized beads.

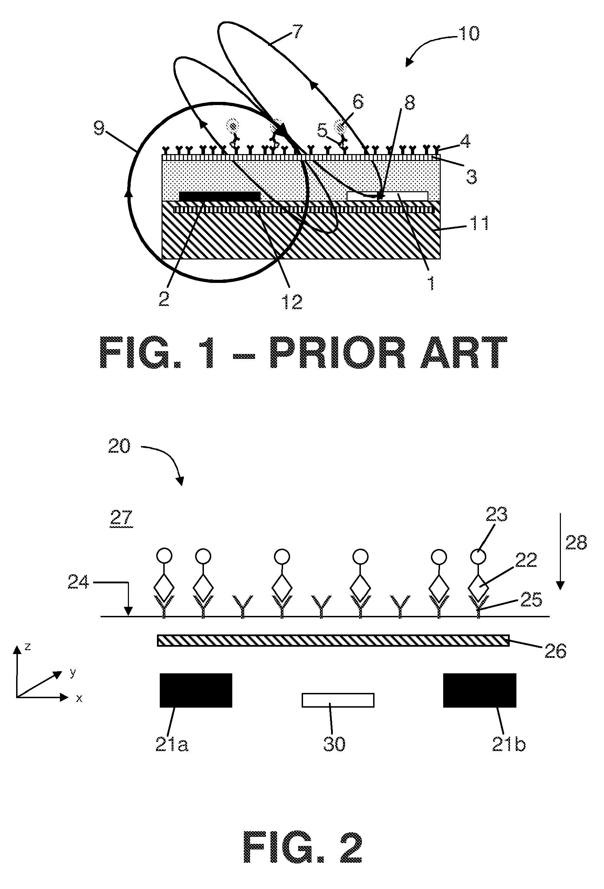

Fig. 1 illustrates the working principle of a magnetoresistive biosensor configuration with integrated magnetic field excitation as presently known in the art. The magnetoresistive biosensor 10 comprises a single GMR strip 1, e.g. with a length of about 100 µm and a width of about 3 µm. With integrated magnetic field excitation is meant that a magnetic field generating means is integrated in the magnetoresistive sensor 10. The magnetoresistive sensor 10 furthermore comprises a current wire 2 as magnetic field generating means. At a surface 3 of the magnetoresistive biosensor 10, a bio-active layer of binding sites 4 are provided to which, for example, target molecules 5 with attached thereto a magnetic bead 6, e.g. a superparamagnetic nanoparticle, can bind. A current flowing through the current wire 2 generates a magnetic field 9 which magnetizes the magnetic bead 6, e.g. superparamagnetic nanoparticle. The magnetic bead 6, e.g. magnetic nanoparticle, develops a magnetic moment m indicated by field lines 7 in Fig. 1. The magnetic moment m then generates dipolar magnetic fields, which have in-plane magnetic field components 8 at the location of the GMR strip 1. Thus, the magnetic bead 6, e.g. magnetic nanoparticle, deflects the magnetic field 9 induced by the current through the current wire 2, resulting in the magnetic field component 8 in the sensitive x-direction of the GMR strip 1, also called x-component 8 of the magnetic field H. The x-component 8 of the magnetic field H is then sensed by the GMR strip 1 and depends on the number Nₙₚ of magnetic beads 6, e.g. superparamagnetic nanoparticles, present at the surface 3 of the magnetoresistive biosensor 10 and on the magnitude of the current in the current wire 2. The magnetoresistive biosensor 10 may be formed on a semiconductor chip, e.g. silicon chip 11, in which electronic circuitry 12 may be embedded.

Biosensors in which a magnetic bead label is bound to a sensor surface 3 are interesting due to the following properties:
- They show a low interference of magnetic beads 6 with complex biological samples and thus provide enhanced sensitivity and robustness.
- The possibility exists to magnetically control the release of beads 6, to concentrate beads 6 on the sensor surface 3 to enhance the binding kinetics, and to remove unbound or weakly bound particles from the sensor surface 3.

After specific binding of magnetic bead labels in a biological assay, it may be important to be able to move and replace the fluid over the sensor surface 3 for further (bio)chemical processing and/or detection, e.g.:
- (bio)chemically cross-linking the labels to the sensor surface 3,
   modifying the labels on the sensor surface 3 for secondary labeling or for signal amplification, and/or
- supplying materials for the generation of a further reaction, e.g. for chemiluminescence detection.

It has been observed that movement or replacement of fluid over the sensor surface 3 can perturb magnetic labels 6 that are specifically bound to the sensor surface 3. The fluid movement or replacement exerts a force on the particles 6, which may be detrimental to the sensitivity and reproducibility of the sensing process. The labels 6 are disturbed by movement of a fluid, and particularly disturbed when a fluid/fluid meniscus passes over the sensor surface 3, e.g. a liquid/liquid or liquid/gas meniscus. An assay that has been found to be particularly sensitive to forces exerted on the beads 6 is a so-called catch assay, in which a bead 6 specifically catches an analyte or target molecule 5 to be detected in solution and thereafter binds to the sensor surface 3.

### SUMMARY OF THE INVENTION

It is an object of embodiments of the present invention to provide a good magnetic sensor device and method for determining the presence and/or amount of target moieties in a sample fluid and/or to a method of manufacturing the sensor device.

The above objective is accomplished by a method and device according to the present invention.

The magnetic sensor device and method according to embodiments of the present invention allow manipulating fluids on a sensor surface while not or substantially not disturbing specific bindings between target moieties labeled with magnetic or magnetizable objects and the sensor surface and/or between specific bindings between the magnetic or magnetizable objects and the target moieties they are attached to. This may increase reliability and accuracy of the magnetic sensor device and method according to embodiments of the invention.

The magnetic sensor device according to embodiments of the present invention shows good sensitivity and is effective.
The magnetic sensor device and method according to embodiments of the present invention may be used in molecular diagnostics, biological sample analysis or chemical sample analysis.

Particular and preferred aspects of the invention are set out in the accompanying independent and dependent claims. Features from the dependent claims may be combined with features of the independent claims and with features of other dependent claims as appropriate and not merely as explicitly set out in the claims.

In a first aspect, a magnetic sensor device is provided for determining the presence and/or amount of target moieties in a sample fluid, the target moieties being labeled with magnetic or magnetizable objects. The magnetic sensor device comprises:
- a sensor surface adapted for binding the labeled target moieties,
- a magnetic field generating means adapted for generating a magnetic retention field for retaining the labeled target moieties to the sensor surface and/or for keeping the magnetic or magnetizable objects bound to the target moieties during sample fluid manipulations, and
- at least one sensor element for providing a sensor signal representative for the presence and/or amount of labeled target moieties bound to the sensor surface.

Because of the magnetic retention field, the labeled target moieties are retained on the sensor surface and/or the magnetic or magnetizable objects are kept bound to the target moieties and thus these bindings are not or substantially not disturbed during fluid manipulation. This leads to a reliable and accurate sensor signal.

The magnetic field generating means for generating a magnetic retention field may be adapted for generating a magnetic field with a magnetic field gradient with a component oriented in a direction toward the sensor surface.

According to embodiments of the invention, the magnetic field generating means for generating a magnetic retention field may be an on-chip magnetic field generating means. In this case, a relatively weak magnetic field may be enough to keep labeled target moieties bound to the sensor surface during fluid manipulations.

The magnetic field generating means for generating a magnetic retention field may be formed by at least one current wire.

According to other embodiments, the magnetic field generating means for generating a magnetic retention field may be an off-chip magnetic field generating means.

The magnetic field generating means for generating a magnetic retention field may be formed by at least one of a permanent magnet, a coil or an electromagnet.

The magnetic sensor device may furthermore comprise a magnetic field generating means adapted for, before binding the labeled target moieties, attracting them toward the sensor surface by generating a magnetic field having a magnetic field gradient.

According to embodiments of the invention, the magnetic field generating means for generating a magnetic retention field may be the same as the magnetic field generating means for attracting the labeled target moieties toward the sensor surface. According to these embodiments, less space is required because only one magnetic field generating means is to be provided.

The magnetic sensor device may furthermore comprise means for applying a stringency force for removing non-specifically bound and non-bound labeled target moieties from the sensor surface. Through this, unbound and/or weakly bound magnetic or magnetizable objects may be removed from the surface such that they cannot bind to the sensor surface during application of the second magnetic field and negatively influence the sensor signal.

The means for applying a stringency force may be formed by magnetic means, acoustic means, chemical means or optical means.

The at least one sensor element may be a GMR element.
According to other embodiments the at least one sensor element may be an optical sensor element.

According to embodiments of the invention, the magnetic sensor device may be a biosensor.

The present invention also provides the use of the magnetic sensor device according to embodiments of the invention in molecular diagnostics, biological sample analysis or chemical sample analysis.

In a further aspect, the present invention provides a magnetic sensor chip comprising at least one magnetic sensor device according to embodiments of the present invention.

The magnetic sensor chip may be a biochip.

In still a further aspect of the present invention, a method is provided for determining the presence and/or amount of target moieties in a sample fluid, the target moieties being labeled with magnetic or magnetizable objects. The method comprises:
- providing a first sample fluid with labeled target moieties to a magnetic sensor device,
- allowing the labeled target moieties to bind to a surface of the magnetic sensor device,
- removing unbound or non-specifically bound magnetic or magnetizable objects from the sensor surface,
- applying a magnetic retention field for retaining the labeled target moieties to the sensor surface and/or for keeping the magnetic or magnetizable objects bound to the target moieties while applying fluid manipulations,
- measuring a sensor signal by means of at least one sensor element, and
- from the measured sensor signal determining the presence and/or amount of labeled target moieties bound to the sensor surface.

According to embodiments of the invention, applying a magnetic retention field may be performed by sending a current through at least one on-chip current wire.

According to other embodiments, applying a magnetic retention field may be performed by means of one of a permanent magnetic, a coil or an electromagnet under the magnetic sensor device.

According to embodiments of the invention, providing a first sample fluid with labeled target moieties to a sensor device may be performed by:
- providing the first sample fluid comprising target moieties to the magnetic sensor device, and
- adding magnetic or magnetizable objects to the first sample fluid such that they can bind to the target moieties.

According to other embodiments of the invention, providing a first sample fluid with labeled target moieties to a sensor device may be performed by:
- adding magnetic or magnetizable objects to the first sample fluid such that they can bind to the target moieties, and
- providing the first sample fluid comprising the labeled target moieties to the magnetic sensor device.

The method may furthermore comprise, before applying the magnetic retention field, applying a stringency force for removing non-specifically bound and non-bound labeled target moieties from the sensor surface.

The stringency force may be applied by magnetic means, acoustic means, chemical means or optical means.

Applying fluid manipulations may be performed by replacing the first sample fluid by a second fluid.

The present invention also provides the use of the method according to embodiments of the invention in molecular diagnostics, biological sample analysis or chemical sample analysis.

The present invention also provides a controller for controlling actuation of a magnetic attraction field generating means or of a magnetic retention field generating means of a magnetic sensor device. The controller comprises a control unit for controlling a current source for sending a current through at least one current wire of the magnetic attraction field generating means and the magnetic retention field generating means and for controlling a switch controller for controlling flowing of the current through the at least one current wire of the magnetic attraction field generating means or through the at least one current wire of the magnetic retention field generating means.

The present invention, in aspects thereof, furthermore provides a computer program product for performing, when executed on a computing means, a method according to embodiments of the invention and a machine readable data storage device for storing the computer program product according to embodiments of the invention.

The present invention also provides transmission of the computer program product according to embodiments of the invention over a local or wide area telecommunications network.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### BRIEF DESCRIPTION OF THE DRAWINGS

Fig. 1 illustrates the principle of a known magnetic biosensor.
Fig. 2 illustrates a magnetic sensor device according to an embodiment of the present invention.
Fig. 3 illustrates a magnetic sensor device according to an embodiment of the present invention.
Fig. 4 illustrates a magnetic sensor device according to an embodiment of the present invention.
Fig. 5 illustrates a biochip comprising magnetic sensor devices according to embodiments of the present invention.
Fig. 6 schematically illustrates a system controller for use with a magnetic sensor device according to embodiments of the present invention.
Fig. 7 is a schematic representation of a processing system as can be used for performing a method according to embodiments of the present invention.

In the different figures, the same reference signs refer to the same or analogous elements.

### DETAILED DESCRIPTION OF THE EMBODIMENTS

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes.

Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the term under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect of the invention, a magnetic sensor device is provided for determining the presence and/or amount of target moieties in a sample fluid, the target moieties being labeled with magnetic or magnetizable objects. The magnetic sensor device comprises:
- a sensor surface adapted for binding the labeled target moieties,
- a magnetic field generating means adapted for generating a magnetic retention field for retaining the labeled target moieties to the sensor surface and/or for keeping the magnetic or magnetisable objects bound to the target moieties during sample fluid manipulations, and
- at least one sensor element for providing a sensor signal representative for the presence and/or amount of labeled target moieties bound to the sensor surface.

The adaptation of the magnetic field generating means may comprise a controller for controlling the magnetic field generating means so that the labeled target moieties are retained to the sensor surface during sample fluid manipulations and/or for keeping the magnetic or magnetisable objects bound to the target moieties during sample fluid manipulations.

The magnetic sensor device according to embodiments of the present invention allows manipulating fluids on a sensor surface while not or substantially not disturbing specific bindings between target moieties labeled with magnetic or magnetizable objects and the sensor surface and/or between specific bindings between the magnetic or magnetizable objects and the target moieties they are attached to. This may increase reliability and accuracy of the magnetic sensor device according to embodiments of the invention.

The magnetic sensor device according to embodiments of the present invention shows good sensitivity and is effective.

The magnetic sensor device according to embodiments of the present invention may be used in molecular diagnostics, biological sample analysis or chemical sample analysis.

According to embodiments of the invention, the surface of the magnetic sensor device may be modified by a coating which is designed to attract certain target moieties or may be modified by attaching molecules, also referred to as target homologues or target probes, to it which are suitable to bind the target moieties which are possibly present in the sample fluid to be tested. In this way the surface of the magnetic sensor device, or at least part thereof, is activated with such binding molecules to form specific binding sites to enable immobilisation of target moieties. Such binding molecules are known to the skilled person and may include proteins, antibodies, nucleic acids (e.g. DNA, RNA), peptides, oligo- or polysaccharides or sugars, small molecules, hormones, drugs, metabolites, cells or cell fractions, tissue fractions, .... Such molecules may be attached to the sensor substrate surface by means of spacer or linker molecules. Binding sites on sensor substrate surfaces can also be provided with molecules in the form of organisms (e.g. viruses or cells) or fractions of organisms (e.g. tissue fractions, cell fractions, membranes).

Target moieties are to be detected in a sample fluid, which can be the original sample or can already have been processed before insertion into the sensor device (e.g. diluted, digested, degraded, biochemically modified, filtered, dissolved into a buffer). The original fluids can be, for example, biological fluids such as saliva, sputum, blood, blood plasma, cells, interstitial fluid or urine, or other fluids such as drinking fluids, environmental fluids, or a fluid that results from sample pre-treatment. The fluid can, for example, comprise elements of solid sample material, e.g. from biopsies, stool, food, feed, environmental samples.

The present invention will be described by means of the magnetic or magnetizable objects being magnetic particles such as beads. This is only for the ease of explanation and is not intended to limit the invention in any way. The present invention also applies for a magnetizable object being a magnetic rod, a string of magnetic particles, or a composite particle, e.g. a particle containing magnetic as well as optically-active material, or magnetic material inside a non-magnetic matrix. In the following the term "magnetic particles" will be used and this is intended to include both magnetizable as well as (permanent) magnetic objects.

Fig. 2 illustrates a magnetic sensor device 20 according to an embodiment of the present invention. According to the present embodiment, the magnetic sensor device 20 may comprise a magnetic field generating means 21a, 21b for generating a magnetic field having a magnetic field gradient for attracting target moieties 22 to be detected in a sample fluid which are labeled with magnetic particles 23 towards a sensor surface 24 and allowing the attracted labeled target moieties 22, 23 to bind to target probes 25 present on the sensor surface 24. It is known from experimental data that a magnetic field of ~80000 A/m, for example is able to attract all magnetic particles 23 present in the sample fluid to the sensor surface 24. This magnetic field will also be referred to as magnetic attraction field. In magnetic sensor devices 20, and especially in biosensors, once the labeled target moieties 22, 23 are bound to the sensor surface 24, different fluid manipulations may be required, such as for example replacing the sample fluid by another fluid, e.g. comprising magnetic particles 23 for binding to the bound labeled target moieties 22, 23 for amplifying the resulting sensor signal. These fluid manipulations may disturb the binding between the labeled target moieties 22, 23 on the one hand and the target probes 25 on the sensor surface 24 on the other hand and/or between the magnetic particles 23 on the one hand and the target moieties 22 on the other hand, thereby rendering the sensor signal unreliable. In the further description, with fluid manipulation is meant any movement of the sample fluid or any other fluid which could have an influence on the binding of the labeled target moieties 22, 23 to the target probes 25 on the sensor surface 24 and/or of the magnetic particles 23 to the target moieties 22. Therefore, the magnetic sensor device 20 according to the present invention comprises magnetic field generating means 26 adapted for generating a magnetic retention field for retaining the magnetic particles 23 and thus the target moieties 22 labeled with these magnetic particles 23 to the sensor surface 24. The magnetic retention field generating means 26 may be controlled by a controller, so that the labeled target moieties are retained to the sensor surface and/or that the magnetic or magnetisable objects are kept bound to the target moieties during sample fluid manipulations. The generated magnetic retention field can have in-plane as well as out-of-plane components. With in-plane components is meant, when the sensor surface 24 is lying in a plane, magnetic field components lying in a plane substantially parallel to the plane of the sensor surface 24. With out-of-plane components is meant, when the sensor surface 24 is lying in a plane, magnetic field components lying in a plane substantially perpendicular to the plane of the sensor surface 24. The applied magnetic retention field should be such that it has a magnetic field gradient with a strong out-of-plane component, i.e. a component oriented from the sample fluid 27 or any other fluid towards the sensor surface 24. The required field gradient depends on:
(i) the required retention force (which depends on the intended fluid manipulation steps and on the size of the magnetic particles 23),
(ii) the magnetic susceptibility of the magnetic particles 23, and
(iii) the size of the magnetic field.

This strong out-of-plane component generates a retention force on the magnetic particles 23. Hence, due to the magnetic field gradient, the magnetic particles 23 experience a retention force toward the sensor surface 24 (indicated with arrow 28) and are thus less prone to displacement or detachment by the fluid manipulations. As a consequence, bindings between the labeled target moieties 22, 23 and the sensor surface 24 and/or between the magnetic particles 23 and the target moieties 22 are not or substantially not disturbed, which leads to a reliable and accurate sensor signal which is representative for the amount of labeled target moieties 22, 23 bound to the sensor surface 24.

The magnetic field generating means 26 for generating the retention field may be an internal or on-chip magnetic field generating means and may, according to the present embodiment, be formed by a current wire 26. The current wire 26 may be located in between the sensor surface 24 and the magnetic field generating means 21a, 21b for generating the magnetic attraction field. An advantage hereof is that a relatively weak magnetic field may be sufficient to retain the labeled target moieties 22, 23 to the sensor surface 24 and/or to keep the magnetic particles 23 bound to the target moieties 22. However, according to other embodiments, the current wire 26 may also be located further away from the sensor surface 24, for example under the magnetic field generating means 21a, 21b for generating the magnetic attraction field, rather than in between the sensor surface 24 and the magnetic field generating means 21a, 21b for generating the magnetic attraction field (not illustrated in the drawings).

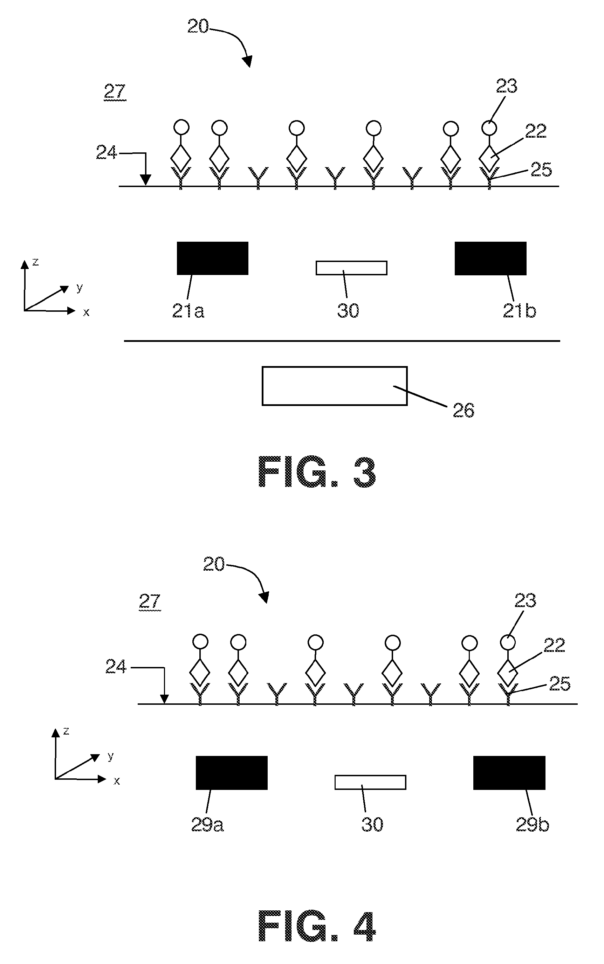

According to other embodiments of the invention, the magnetic field generating means 26 for generating the retention field may be an external or off-chip magnetic field generating means and may be formed by a permanent magnet (as illustrated in Fig. 3), a coil or an electromagnet which may be positioned under the magnetic sensor device 20.

According to still further embodiments of the invention, the magnetic field generating means for generating the magnetic attraction field and the magnetic field generating means 26 for generating the magnetic retention field may be the same. This is illustrated in Fig. 4. The current wires 29a, 29b may function both as magnetic attraction field generating means and magnetic retention field generating means. By sending a first current through the current wires 29a, 29b labeled target moieties 22, 23 are attracted toward the sensor surface 24 and can bind to the target probes 25 present on the sensor surface 24. The first current is then switched off. Non-specifically or weakly bound magnetic particles 23 may be removed from the sensor surface 24 in order to decrease background signals. This may, for example, be done by applying a stringency force (see further). When fluid manipulations are to be applied, a second current may be sent through the current wires 29a, 29b for generating the retention field. In this way, the binding between the labeled target moieties 22, 23 and target probes 25 on the sensor surface 24 and/or between the magnetic particles 23 and the target moieties 22 is not or substantially not disturbed during fluid manipulation. According to embodiments of the invention, the first current for generating a magnetic field for attracting the magnetic particles 23 to the sensor surface 24 may be, but not necessarily is, an AC current, while the current for generating the retention magnetic field may be, but not necessarily is, a DC current.

As can be seen from Fig. 2 to Fig. 4, the magnetic sensor device 20 may furthermore comprise a sensor element 30 for sensing the presence of magnetic particles 23 at the sensor surface 24 and for generating a sensor signal representative for the amount of magnetic particles 23 present at the sensor surface 24. According to embodiments of the invention, the sensor element 30 may be a GMR element. This is, however, not limiting the invention in any way. The present invention may also be applied to magnetic sensor devices 20 comprising any sensor element 30 suitable for detecting the presence or determining the amount of magnetic particles 23 on or near a sensor surface 24 based on any property of the particles 23. For example, detection of the magnetic particles 23 may be done by means of magnetic methods (e.g. magnetoresistive sensor elements, hall sensors, coils), optical methods (e.g. imaging fluorescence, chemiluminescence, absorption, scattering, surface plasmon resonance, Raman, frustrated total internal reflection, ...), sonic detection (e.g. surface acoustic wave, bulk acoustic wave, cantilever, quartz crystal, ...), electrical detection (e.g. conduction, impedance, amperometric, redox cycling), ....

In a second aspect of the invention, a method is provided for determining the presence and/or amount of target moieties 22 in a sample fluid 27, the target moieties 22 being labeled with magnetic particles 23. The method comprises, in the following sequence:
- providing a first sample fluid 27 with labeled target moieties 22, 23 to a magnetic sensor device 20,
- allowing the labeled target moieties 22, 23 to bind to a surface 24 of the magnetic sensor device 20,
- removing unbound or non-specifically bound magnetic or magnetizable objects 23 from the sensor surface 24,
- applying a magnetic retention field for retaining the labeled target moieties 22, 23 to the sensor surface 24 and/or for keeping the magnetic or magnetizable objects 23 bound to the target moieties 22 while applying fluid manipulations,
- measuring a sensor signal by means of at least one sensor element 30, and
- from the measured sensor signal determining the presence and/or amount of labeled target moieties 22, 23 bound to the sensor surface 24.

Providing the sample fluid 27 with labeled target moieties 22, 23 to the magnetic sensor device 20 can be done in different ways. According to embodiments of the invention, a sample fluid 27 comprising target moieties 22 to be detected may be provided to the magnetic sensor device 20. Then, magnetic particles 23 may be added to the sample fluid 27 such that they can bind to the target moieties 22. According to other embodiments, magnetic particles 23 may, outside the magnetic sensor device 20, be added to the sample fluid 27 comprising the target moieties 22 to be detected such that they can bind to the target moieties 22. The sample fluid 27 comprising labeled target moieties 22, 23 may then be provided to the magnetic sensor device 20.

Allowing the labeled target moieties 22, 23 to bind to the sensor surface 24 may preferably be done by switching on the magnetic attraction field generating means hereby generating a magnetic field for attracting the labeled target moieties 22, 23 toward the sensor surface 24. In the examples of Fig. 2 to Fig. 4 the magnetic attraction field generating means is formed by current wires 21a, 21b or 29a, 29b. Hence, by sending a current through the current wires 21 a, 21b or 29a, 29b, the labeled target moieties 22, 23 may be attracted toward the sensor surface 24 and may be allowed to bind to the target probes 25 present on the sensor surface 24. According to other embodiments, however, allowing the labeled target moieties 22, 23 to bind to the sensor surface 24 may be done by incubating the sample fluid for some time such that the labeled target moieties 22, 23 can sink down toward the sensor surface 24 under gravity. This, however, takes more time than when an attraction field, in particular for example a magnetic attraction field, is generated. An advantage of using the gravity is that no attraction field generating means, in particular no magnetic field generating means 21 a, 21 b, is required.

According to embodiments of the invention, prior to application of the retention field, preferably a stringency force may be applied to the labeled target moieties 22, 23. This may be done by any suitable stringency force generating means, for example by magnetic means, acoustic means, chemical means or optical means. The stringency force may remove unbound and weakly or non-specifically bound magnetic particles 23 from the sensor surface 24 and from the vicinity of the sensor surface 24 because the presence of such unbound and weakly or non-specifically bound magnetic particles 23 at the sensor surface 24 can perturb the sensor signal in two ways:
- Unbound and weakly or non-specifically bound magnetic particles 23 may become bound to the sensor surface 24 in a non-specific way when they are exposed to the sensor surface 24 for a long time. This can in particular happen during application of the retention force. This may result in an unwanted background signal in the measured sensor signal.
- Upon application of the retention field, unbound or weakly bound magnetic particles 23 can be attracted toward and become bound to the specifically bound particles due to magnetic dipole-dipole interactions. This can perturb the retention force on the specifically bound magnetic particles 23 and can deteriorate the effectiveness and reliability of the magnetic sensor device 20.
   According to embodiments of the invention, the stringency field for removing unbound and weakly or non-specifically bound magnetic particles 23 from the sensor surface 24 may have a magnitude of, for example, 175000 A/m.

In some cases it may be preferred or required to strongly fix the specifically bound labeled target moieties 22, 23 to the sensor surface 24. This may, for example, be the case when very strong fluid manipulations have to be performed such as e.g. when drying the sensor surface 24 for storage purposes. One way to obtain such strong fixation may be by applying a very strong retention force. Strong retention forces may generate additional bindings between the labeled target moieties 22, 23 and the sensor surface 24 and between target moieties 22 and magnetic particles 23, so that the labeled target moieties 22, 23 will be strongly fixed to the sensor surface 24 such that, during fluid manipulations, these labeled target moieties 22, 23 are kept to the sensor surface 24. Alternatively or additionally, the fixation of labeled target moieties 22, 23 may be achieved by, for example, chemical linking, e.g. cross-linking of the labeled target moieties 22, 23 to the target probes 25 on the sensor surface 24. The chemical linking process may be enhanced by radiation (e.g. optical radiation), by temperature, or by the application of a current or voltage to an electrode integrated in a sensor substrate or located near to the sensor surface 24.

The applied retention force may be different in a non-magnetic fluid than in a fluid with magnetic elements Since biological and biochemical fluids are mostly not magnetic or only very weakly magnetic, the applied retention forces may be generally well controllable.

The method according to embodiments of the invention may be combined with measures to reduce fluidic forces on the sensor surface 24. Forces of a fluid on magnetic particles 23 can be of a physical nature (e.g. hydrodynamic drag), but can also be of a physico-chemical nature (e.g. bond weakening due to chemical composition of the fluid). Reducing the fluidic forces may, for example, be done by applying shear-reducing or meniscus-pinning structures on the sensor surface 24, e.g. small mechanical protrusions extending from the sensor surface 24 in a direction substantially perpendicular to the plane of the sensor surface 24, hereby forming cavity-like areas in between the protrusions. Preferably the labeled target moieties 22, 23 may be bound in the cavity-like areas between the protrusions, which can be achieved by attraction of labeled target moieties 22, 23 into the cavities during incubation, preferably by applying magnetic forces, and/or by only providing biochemical binding areas comprising target probes 25 for binding the labeled target moieties 22, 23 inside the cavities.

Hereinafter, two examples of the method according to embodiments of the present invention will be described. These examples are only for the ease of understanding and are not intended to limit the invention in any way.

A first example describes a sandwich assay with magnetic-particle labels, in which the labels are detected by chemiluminescence. Two types of antibodies are used:
- antibody-1, which has particular affinity to epitope-1 of the target moieties 22 to be detected in the sample fluid 27, and
- antibody-2, which has particular affinity to epitope-2 of the target moieties 22 to be detected in the sample fluid 27.

The two epitopes, i.e. an antigenic site on a protein against which an antibody reacts, are selected such that a sandwich format is possible, i.e. the target moiety 22 to be detected can be bound simultaneously by antibody-1 and antibody-2. The sensor surface 24 is coated with antibody-1 and the magnetic particles 23 are coated with antibody-2.

According to one example, the catch-type assay may be performed as follows:
- The sample fluid 27 comprising the target moieties 22 to be detected is inserted into a reaction chamber of the magnetic sensor device 20.
- Magnetic particles 23 coated with antibody-2 are added to the sample fluid 27.
- Incubation for some time, e.g. for up to 60 minutes, so that target moieties 22 can bind to the magnetic particles 23.
- A magnetic field gradient is applied for attracting the labeled target moieties 22, 23 toward the sensor surface 24 and to concentrate them near the sensor surface 24. For applying the magnetic field gradient a Magnetic fisher and a Dynal MPC®-96B (in which a residual magnetic flux density of 2500 Gauss (0.25 Tesla) may be obtained) were used.
- Incubation for some time so that labeled target moieties 22 are able to bind to the sensor surface 24 via a molecular sandwich format. Incubation can, for example, be done by keeping the magnetic field on for e.g. 10 minutes, or can be done in steps, e.g. keeping the magnetic field on for 1 minute and off for 1 minute and repeating these two steps e.g. five times.
- A magnetic stringency field is applied to remove unbound and weakly bound labeled target moieties 22 from the sensor surface 24. For applying the magnetic stringency filed a magnetic fisher and a magnetic separator for 96-well plates from Retro-Tech Gmbh (comparable with a Dyal MPC®-96S in which a residual magnetic flux density of 3500 Gauss (0.35 Tesla) may be obtained) were used. The magnetic field for removing unbound and weakly bound labeled target moieties 22 from the sensor surface 24 according to this example may have a magnitude of 175000 A/m.
- A retention field is applied, and the sample fluid 27 in the reaction chamber replaced with a solution with a known target moiety concentration. For applying the retention field a Dynal MPC®-96B was used in which a residual magnetic flux density of 2500 Gauss (0.25 Tesla) may be obtained.
- Incubation for some time , e.g. for up to 20 minutes, so that the target moieties can couple to free antibody-1 on the sensor surface 24 as well as to free antibody-2 on the magnetic particles 23.
- A retention field is applied and the unbound target moieties are washed away. In other words, excess of target moieties labeled with magnetic particles 23 but not bound to free antibody-1 on the sensor surface 24 are removed. For applying the retention field a Dynal MPC®-96B was used in which a residual magnetic flux density of 2500 Gauss (0.25 Tesla) may be obtained.
- A retention field is applied and a solution with tagged-antibody-1 is added, the tag can for example be biotin or a chemiluminescence moiety. For applying the retention field a Dynal MPC®-96B was used in which a residual magnetic flux density of 2500 Gauss (0.25 Tesla) may be obtained.
- Incubation for some time, e.g. for 10 minutes, so that tagged-antibody-1 can bind to target moieties 22 bound to magnetic particles 23.
- A retention field is applied and unbound tagged-antibody-1 are washed away. For applying the retention field a Dynal MPC®-96B was used in which a residual magnetic flux density of 2500 Gauss (0.25 Tesla) may be obtained.
- Chemiluminescence from the tagged-antibody-1 is developed and measured. This signal is a measure for the concentration of magnetic particles 23 on the sensor surface 24.
- A second example describes an assay in which the magnetic signal is amplified. This example may comprise the following steps:

- An assay is performed with magnetic particles 23 as labels, e.g. a sandwich assay or a competition assay (see above).
- A magnetic field is applied to remove unbound magnetic particles 23. Specifically bound labeled target moieties 22, 23 hereby remain on the sensor surface 24.
- A retention field is applied and the non-magnetic components, e.g. unbound target moieties 22 and unreacted assay reagents, are washed away.
- A solution is added comprising additional magnetic particle labels that can bind to labels that were already bound to the sensor surface 24.
- A magnetic field is applied to remove unbound magnetic particles 22.
- The signal is detected.

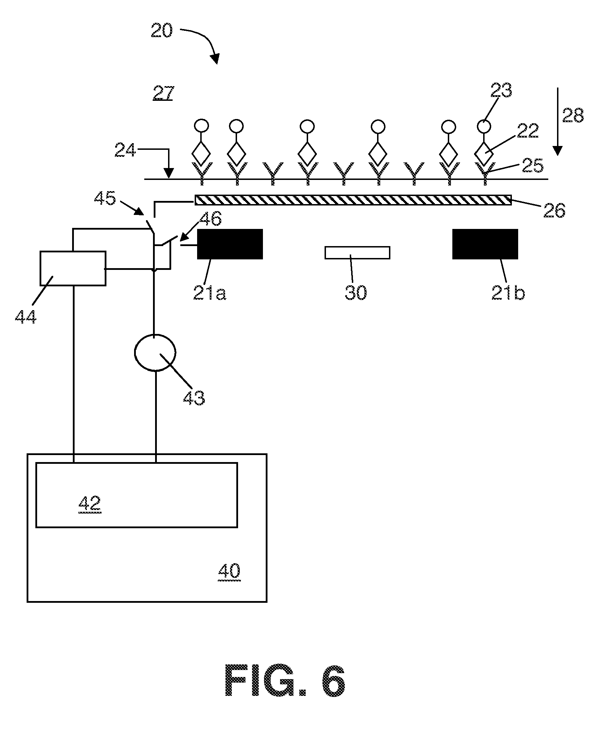

In a further aspect, the present invention also provides a system controller 40 for use with a magnetic sensor device 20 for controlling switching on and off of the magnetic attraction and/or retention field generating means in the magnetic sensor device 20 according to embodiments of the invention. The system controller 40, which is schematically illustrated in Fig. 6 for the embodiment illustrated in Fig. 2, may control the overall operation of the magnetic sensor device 20 for controlling switching on and off of the magnetic attraction and retaining field generating means. The system controller 40 according to the present aspect may comprise a control unit 42 for controlling a current source 43 and for controlling a switch controller 44. Controlling the switch controller 44 may be such that current from the current source 43 is sent either through switch 45 to the magnetic retention field generating means 26 or through switch 46 to magnetic attraction field generating means 21a, 21b. It is clear for a person skilled in the art that the system controller 40 may comprise other control units for controlling other parts of the magnetic sensor device 20; however, such other control units are not illustrated in Fig. 6.

The system controller 40 may include a computing device, e.g. microprocessor, for instance it may be a micro-controller. In particular, it may include a programmable controller, for instance a programmable digital logic device such as a Programmable Array Logic (PAL), a Programmable Logic Array, a Programmable Gate Array, especially a Field Programmable Gate Array (FPGA). The use of an FPGA allows subsequent programming of the microfluidic system, e.g. by downloading the required settings of the FPGA. The system controller 40 may be operated in accordance with settable parameters.

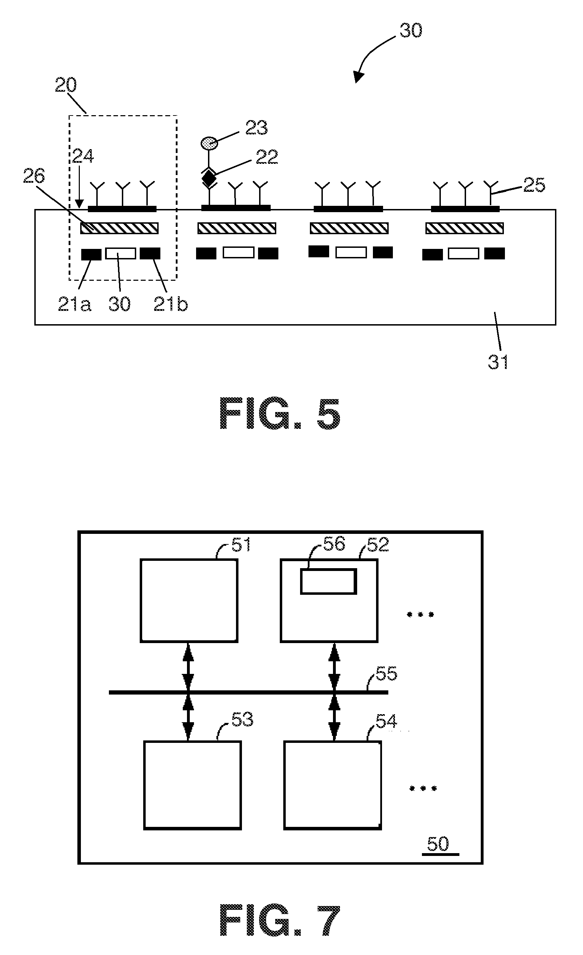

The above-described method embodiments of the present invention may be implemented in a processing system 50 such as shown in Fig. 7. Fig. 7 shows one configuration of processing system 50 that includes at least one programmable processor 51 1 coupled to a memory subsystem 52 that includes at least one form of memory, e.g., RAM, ROM, and so forth. It is to be noted that the processor 51 or processors may be a general purpose, or a special purpose processor, and may be for inclusion in a device, e.g., a chip that has other components that perform other functions. Thus, one or more aspects of the present invention can be implemented in digital electronic circuitry, or in computer hardware, firmware, software, or in combinations of them. The processing system may include a storage subsystem 53 that has at least one disk drive and/or CD-ROM drive and/or DVD drive. In some implementations, a display system, a keyboard, and a pointing device may be included as part of a user interface subsystem 54 to provide for a user to manually input information. Ports for inputting and outputting data, e.g. desired or obtained flow rate, also may be included. More elements such as network connections, interfaces to various devices, and so forth, may be included, but are not illustrated in Fig. 7. The various elements of the processing system 50 may be coupled in various ways, including via a bus subsystem 55 shown in Fig. 7 for simplicity as a single bus, but will be understood to those in the art to include a system of at least one bus. The memory of the memory subsystem 52 may at some time hold part or all (in either case shown as 56) of a set of instructions that when executed on the processing system 50 implement the steps of the method embodiments described herein. Thus, while a processing system 50 such as shown in Fig. 7 is prior art, a system that includes the instructions to implement aspects of the method according to embodiments of the invention is not prior art, and therefore Fig. 7 is not labeled as prior art.

The present invention also includes a computer program product which provides the functionality of any of the methods according to the present invention when executed on a computing device. Such computer program product can be tangibly embodied in a carrier medium carrying machine-readable code for execution by a programmable processor. The present invention thus relates to a carrier medium carrying a computer program product that, when executed on computing means, provides instructions for executing any of the methods as described above. The term "carrier medium" refers to any medium that participates in providing instructions to a processor for execution. Such a medium may take many forms, including but not limited to, non-volatile media, and transmission media. Non volatile media includes, for example, optical or magnetic disks, such as a storage device which is part of mass storage. Common forms of computer readable media include, a CD-ROM, a DVD, a flexible disk or floppy disk, a tape, a memory chip or cartridge or any other medium from which a computer can read. Various forms of computer readable media may be involved in carrying one or more sequences of one or more instructions to a processor for execution. The computer program product can also be transmitted via a carrier wave in a network, such as a LAN, a WAN or the Internet. Transmission media can take the form of acoustic or light waves, such as those generated during radio wave and infrared data communications. Transmission media include coaxial cables, copper wire and fibre optics, including the wires that comprise a bus within a computer.

In a further aspect, the present invention also provides a magnetic sensor chip, e.g. biochip 30, comprising at least one magnetic sensor device 20 according to embodiments of the present invention. Fig. 5 illustrates a biochip 30 according to an embodiment of the present invention. The biochip 30 may comprise at least one, and preferably a plurality of magnetic sensor devices 20 according to embodiments of the present invention integrated in a substrate 31. The term "substrate" may include any underlying material or materials that may be used, or upon which a device, a circuit or an epitaxial layer may be formed. The term "substrate" may include a semiconductor substrate such as e.g. a doped silicon, a gallium arsenide (GaAs), a gallium arsenide phosphide (GaAsP), an indium phosphide (InP), a germanium (Ge), or a silicon germanium (SiGe) substrate. The "substrate" may include, for example, an insulating layer such as a SiO₂ or an Si₃N₄ layer in addition to a semiconductor substrate portion. Thus the term "substrate" also includes glass, plastic, ceramic, silicon-on-glass, silicon-on-sapphire substrates. The term "substrate" is thus used to define generally the elements for layers that underlie a layer or portions of interest. Also the "substrate" may be any other base on which a layer is formed, for example a glass or metal layer.

According to embodiments of the invention a single magnetic sensor device 20 or a multiple of magnetic sensor devices 20 may be integrated on the same substrate 31 to form the biochip 30.

According to the example given in Fig. 5, each of the magnetic sensor devices 20 of the biochip 30 may comprise a separate magnetic attraction field generating means, for example implemented by first and second current wires 21a and 21b. However, according to other embodiments of the invention, also other means rather than current wires 21a, 21b may be applied to generate the magnetic attraction field for attracting labeled target moieties 22, 23 toward the sensor surface 24. Furthermore, the magnetic attraction field generating means 21a, 21b may also comprise another number of current wires. In still alternative embodiments, a single magnetic attraction field generating means may be provided for a plurality, for example all, of the magnetic sensor devices 20 of the biochip 30.

In each magnetic sensor device 20 at least one sensor element 30, for example a GMR element, may be integrated in the substrate 31 to read out the information gathered by the biochip 30, thus for example to read out the presence or absence of target moieties 22 via magnetic particles 23 attached to the target moieties 22, thereby determining or estimating an areal density of the target moieties 22. The magnetic particles 22 are preferably implemented by so called superparamagnetic beads. Binding sites may be provided by e.g. target probes 25 which are able to selectively bind a target moiety 22 are provided to the sensor surface 24.

According to embodiments of the present invention, each magnetic sensor device 20 may comprise a separate magnetic retention field generating means 26 for generating a retention field for keeping the bound labeled target moieties 22, 23 to the sensor surface 24 during fluid manipulations. According to other embodiments, a single magnetic retention field generating means 26 may be provided for a plurality of, optionally for all, magnetic sensor devices 20. According to the example given in Fig. 5, the a magnetic retention field generating means 26 may be implemented by a current wire 26 located in the substrate 31 in between the sensor surface 24 and the magnetic attraction field generating means 21a, 21b and the sensor element 30. However, according to other embodiments, the magnetic retention field generating means 26 may be implemented as described in the above embodiments, and as for example illustrated in Fig. 3 and 4.

The functioning of the biochip 30, and thus also of the magnetic sensor device 20, will be explained hereinafter. Each target probe 25 on the sensor surface 24 may be suitable for binding pre-determined target moieties 22. A sample fluid 27 comprising target moieties 22 to be detected may be presented to or passed over the sensor surface 24 of the biochip 30. If the target probes 25 and the target moieties 22 match, they bind to each other. The superparamagnetic beads 23, or more generally the magnetic particles 23, may be directly or indirectly coupled to the target moieties 22. The superparamagnetic beads 23 allow reading out the information gathered by the biochip 30.

In addition to molecular assays, also larger moieties can be detected, e.g. cells, viruses, or fractions of cells or viruses, tissue extract, etc. Detection can occur with or without scanning of the sensor element 30 with respect to the sensor surface 24.

Measurement data can be derived as an end-point measurement, as well as by recording signals kinetically or intermittently.

The magnetic particles can be detected directly by the sensing method. Alternatively, the magnetic particles 23 can be further processed prior to detection. An example of further processing is that materials are added or that the (bio)chemical or physical properties of the magnetic particles 23 are modified to facilitate detection.

The magnetic sensor device 20, biochip 30 and method according to embodiments of the present invention can be used with several biochemical assay types, e.g. binding/unbinding assay, sandwich assay, competition assay, displacement assay, enzymatic assay, etc.

The magnetic sensor device 20, biochip 30 and method according to embodiments of this invention are suitable for sensor multiplexing (i.e. the parallel use of different sensors and sensor surfaces), label multiplexing (i.e. the parallel use of different types of labels or magnetic or magnetizable objects) and chamber multiplexing (i.e. the parallel use of different reaction chambers).

The magnetic sensor device 20, biochip 30 and method according to embodiments of the present invention can be used as rapid, robust, and easy to use point-of-care biosensors for small sample volumes. The reaction chamber can be a disposable item to be used with a compact reader, containing the one or more magnetic field generating means and one or more detection means. Also, the device, methods and systems of the present invention can be used in automated high-throughput testing. In this case, the reaction chamber may, for example, be a well plate or cuvette, fitting into an automated instrument.

It is to be understood that although preferred embodiments, specific constructions and configurations, as well as materials, have been discussed herein for devices according to the present invention, various changes or modifications in form and detail may be made without departing from the scope of this invention as defined by the appended claims.

## Claims

1. A magnetic sensor device (20) for determining the presence and/or amount of target moieties (22) in a sample fluid (27), the target moieties (22) being labeled with magnetic or magnetizable objects (23), the magnetic sensor device comprising:
- a sensor surface (24) adapted for binding the labeled target moieties (22, 23),
- a magnetic field generating means (26) adapted for generating a magnetic retention field for retaining the labeled target moieties (22, 23) to the sensor surface (24) and/or for keeping the magnetic or magnetizable objects (23) bound to the target moieties (22) during sample fluid manipulations, and
- at least one sensor element (30) for providing a sensor signal representative for the presence and/or amount of labeled target moieties (22, 23) bound to the sensor surface (24).

2. A magnetic sensor device (20) according to claim 1, wherein the magnetic field generating means (26) for generating a magnetic retention field is adapted for generating a magnetic field with a magnetic field gradient with a component oriented in a direction toward the sensor surface (24).

3. A magnetic sensor device (20) according to claim 1 or 2, wherein the magnetic field generating means (26) for generating a magnetic retention field is an on-chip magnetic field generating means.

4. A magnetic sensor device (20) according to claim 3, wherein the magnetic field generating means (26) for generating a magnetic retention field is formed by at least one current wire.

5. A magnetic sensor device (20) according to claim 1 or 2, wherein the magnetic field generating means (26) for generating a magnetic retention field is an off-chip magnetic field generating means.

6. A magnetic sensor device (20) according to claim 5, wherein the magnetic field generating means (26) for generating a magnetic retention field is formed by at least one of a permanent magnet, a coil or an electromagnet.

7. A magnetic sensor device (20) according to any of the previous claims, wherein the magnetic sensor device (20) furthermore comprises a magnetic field generating means (21 a, 21b) adapted for, before binding the labeled target moieties (22, 23), attracting them toward the sensor surface (24).

8. A magnetic sensor device (20) according to claim 7, wherein the magnetic field generating means (26) for generating a magnetic retention field is the same as the magnetic field generating means (21a, 21b) for attracting the labeled target moieties (22, 23) toward the sensor surface (24).

9. A magnetic sensor device (20) according to any of the previous claims, furthermore comprising means for applying a stringency force for removing non-specifically bound and non-bound labeled target moieties (22, 23) from the sensor surface (24).

10. A magnetic sensor device (20) according to claim 9, wherein the means for applying a stringency force is formed by magnetic means, acoustic means, chemical means or optical means.

11. A magnetic sensor device (20) according to any of the previous claims, wherein the at least one sensor element (30) is a GMR element.

12. A magnetic sensor device (20) according to any of claims 1 to 10, wherein the at least one sensor element (30) is an optical sensor element.

13. A magnetic sensor device (20) according to any of the previous claims, wherein the magnetic sensor device (20) is a biosensor.

14. Use of the magnetic sensor device (20) according to any of the previous claims in molecular diagnostics, biological sample analysis or chemical sample analysis.

15. A magnetic sensor chip (30) comprising at least one magnetic sensor device (20) according to any of claims 1 to 13.

16. A magnetic sensor chip (30) according to claim 15, wherein the magnetic sensor chip (30) is a biochip.

17. A method for determining the presence and/or amount of target moieties (22) in a sample fluid (27), the target moieties (22) being labeled with magnetic or magnetizable objects (23), the method comprising:
- providing a first sample fluid (27) with labeled target moieties (22, 23) to a magnetic sensor device (20),
- allowing the labeled target moieties (22, 23) to bind to a surface (24) of the magnetic sensor device (20),
- removing unbound or non-specifically bound magnetic or magnetizable objects (23) from the sensor surface (24),
- applying a magnetic retention field for retaining the labeled target moieties (22, 23) to the sensor surface (24) and/or for keeping the magnetic or magnetizable objects (23) bound to the target moieties (22) while applying fluid manipulations,
- measuring a sensor signal by means of at least one sensor element (30), and
- from the measured sensor signal determining the presence and/or amount of labeled target moieties (22, 23) bound to the sensor surface (24).

18. A method according to claim 17, wherein applying a magnetic retention field is performed by sending a current through at least one on-chip current wire (21a, 21b).

19. A method according to claim 17, wherein applying a magnetic retention field is performed by means of one of a permanent magnetic, a coil or an electromagnet under the magnetic sensor device (20).

20. A method according to any of claims 17 to 19, wherein providing a first sample fluid (27) with labeled target moieties (22, 23) to a sensor device (20) is performed by:
- providing the first sample fluid (27) comprising target moieties (22) to the magnetic sensor device (20), and
- adding magnetic or magnetizable objects (23) to the first sample fluid (27) such that they can bind to the target moieties (22).

21. A method according to any of claims 17 to 19, wherein providing a first sample fluid (27) with labeled target moieties (22, 23) to a sensor device (20) is performed by:
- adding magnetic or magnetizable objects (23) to the first sample fluid (27) such that they can bind to the target moieties (22), and
- providing the first sample fluid (27) comprising the labeled target moieties (22, 23) to the magnetic sensor device (20).

22. A method according to any of claims 17 to 21, wherein the method furthermore comprises, before applying the magnetic retention field, applying a stringency force for removing non-specifically bound and non-bound labeled target moieties (22, 23) from the sensor surface (24).

23. A method according to claim 22, wherein the stringency force is applied by magnetic means, acoustic means, chemical means or optical means.

24. A method according to any of claims 17 to 23, wherein applying fluid manipulations is performed by replacing the first sample fluid (27) by a second fluid.

25. Use of the method according to any claims 17 to 24 in molecular diagnostics, biological sample analysis or chemical sample analysis.

26. A controller (40) for controlling actuation of a magnetic attraction field generating means (21a, 21b) or of a magnetic retention field generating means (26) of a magnetic sensor device (20), the controller comprising:
- a control unit (42) for controlling a current source (43) for sending a current through at least one current wire (21 a, 21b) of the magnetic attraction field generating means (21a, 21b) and the magnetic retention field generating means (26) and for controlling a switch controller (44) for controlling flowing of the current through the at least one current wire (21 a, 21 b) of the magnetic attraction field generating means (21 a, 21 b) or through the at least one current wire of the magnetic retention field generating means (26).

27. A computer program product for performing, when executed on a computing means, a method as in any of claims 17 to 24.

28. A machine readable data storage device for storing the computer program product of claim 27.

29. Transmission of the computer program product of claim 27 over a local or wide area telecommunications network.
